# EUROPÄISCHE PATENTANMELDUNG

(11) **EP 1 191 107 A2**
(43) Veröffentlichungstag der Anmeldung: **27.03.2002**
(21) Anmeldenummer: 01250300.9
(22) Anmeldetag: 21.08.2001
(51) Int. Cl.: C12Q 1/68, G01N 33/53

(54) **Methode zur in vitro Diagnostik von Endometriose**

(30) Priorität: 25.09.2000 DE 10048633
(71) Anmelder: Schering Aktiengesellschaft, 13353 Berlin (DE)
(72) Erfinder: Hess-Stumpp, Holger, Dr., 13505 Berlin (DE); Haendler, Bernard, Dr., 13465 Berlin (DE); Krätzschmar, Jörn, Dr., 13409 Berlin (DE); Kreft, Bertholt, Dr., 13158 Berlin (DE); Winterhager, Elke, Prof.Dr., 45259 Essen (DE); Regidor, Pedro, PD Dr., 45133 Essen (DE); Scotti, Simone, Dr., 45529 Hattingen (DE)

(57) **Zusammenfassung**

Die Erfindung betrifft eine Methode zur Diagnose von Endometriose wobei die Menge an Genprodukt von mindestens einem Gen aus der Gruppe bestehend aus Fibronectin, Insulin-like growth factor binding protein-2, Transmembrane receptor PTK7, Platelet-derived growth factor receptor alpha, Collagen type XVIII alpha 1, Subtilisin-like protein (PACE4), Laminin M chain (Merosin), Elastin, Collagen type IV alpha 2, p27 interferon alpha-inducible gene, Reticulocalbin, Aldehyde Dehydrogenase 6, Gravin, Nidogen und Phospholipase C Epsilon in einer Patientinnenprobe bestimmt wird.

## Beschreibung

Die Erfindung betrifft eine Methode zur in vitro Diagnostik von Endometriose.

Endometriose ist eine der häufigsten gynäkologischen Erkrankungen, von der schätzungsweise 5-10% aller Frauen im reproduktionsfähigen Alter betroffen sind (Sillem, M. 1998; Programmed® 23, Suppl. 1, 1-28). Sie ist gekennzeichnet durch das Vorkommen von Endometriumsgewebe außerhalb der physiologischen Schleimhautauskleidung des Uterus. Neben Schmerzen und zahlreichen anderen Symptomen sind viele Endometriosepatientinnen steril, und ein großer Teil der IVF-Patientinnen (IVF - in vitro Fertilisation) leidet an Endometriose (Adamson, G.D. 1997; Sem. Reprod. Biol. 15, 263-271). In jüngster Zeit mehren sich Publikationen, die für eine genetische Prädisposition bei der Entwicklung einer Endometriose sprechen (Kennedy, S. 1997; Sem. Reprod. Biol. 15, 309-318). So wurde der Verlust von Tumorsuppressormolekülen sowie familiäre Häufungen bei EndometriosePatientinnen beschrieben.

Zur Zeit wird die Endometriose mit Hilfe der Laparoskopie diagnostiziert. Dies ist eine invasive Methode, die häufig zu Komplikationen führt (Chapron C. et al. 1998; Hum. Reprod. 13, 867-872; Jansen F. W. et al, 1997; Br. J. Obstet. Gynecol. 104, 595-600). Sie wird unter Narkose durchgeführt und setzt einen voll eingerichteten Operationssaal voraus.

Daher besteht ein Bedarf an neuen Diagnostikmethoden. Wünschenswert wäre eine Methode, die für die Patientinnen weniger belastend wäre und die vom behandelnden Arzt durchgeführt werden könnte.

Dieses Problem wird erfindungsgemäß gelöst durch die Identifizierung von bei der Endometriose differentiell regulierten Genen und die Bereitstellung einer Methode zur Detektion ihrer Genprodukte.

Die Erfindung betrifft eine Methode zur in vitro Diagnose von Endometriose wobei die Menge an Genprodukt von mindestens einem Gen aus der Gruppe bestehend aus Fibronectin, Insulin-like growth factor binding protein-2, Transmembrane receptor PTK7, Platelet-derived growth factor receptor alpha, Collagen type XVIII alpha 1, Subtilisin-like protein (PACE4), Laminin M chain (Merosin), Elastin, Collagen type IV alpha 2, p27 interferon alpha-inducible gene, Reticulocalbin, Aldehyde Dehydrogenase 6, Gravin, Nidogen und Phospholipase C Epsilon in einer Patientinnenprobe bestimmt wird und mit der Menge an diesem Genprodukt in einer Kontrollprobe verglichen wird, wobei eine geringere Menge an diesem Genprodukt auf das Vorliegen einer Endometriose hinweist.

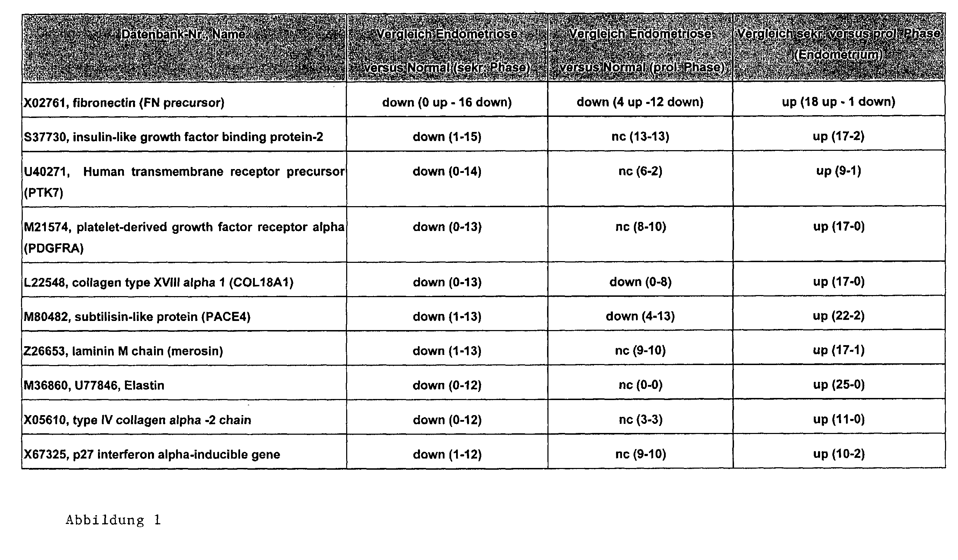

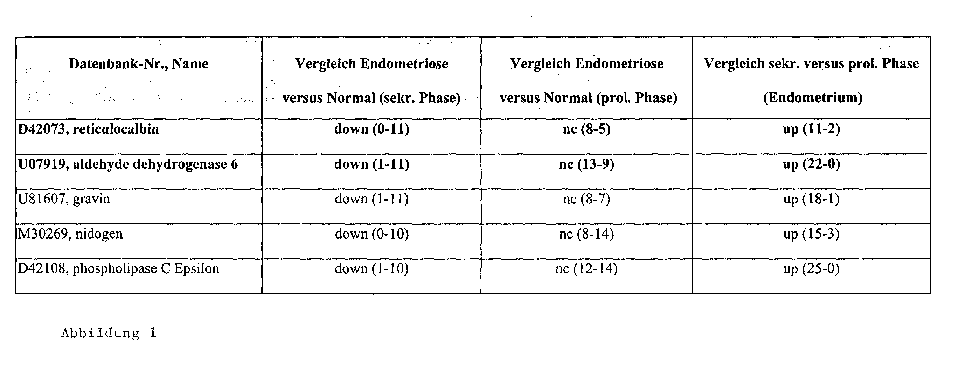

Die Gruppe der Gene wird in Abbildung 1 näher beschrieben. Die Expressionsstärke, d.h. die Menge des Genproduktes von mindestens einem der in Abbildung 1 genannten Gene in einer Patientinnenprobe wird bestimmt und mit der aus einer Kontrollprobe (Frau ohne Endometriose) verglichen. Die zu vergleichenden Proben müssen beide aus der sekretorischen Phase, also aus dem Bereich Tag 15-28 nach der letzten Menstruation stammen. Eine verminderte Expressionsstärke von mindestens einem der o.g. Gene in der Patientinnenprobe deutet auf das Vorliegen einer Endometriose hin.

Eine Patientinnenprobe kann eine Probe vom Endometriumgewebe, Peritonealflüssigkeit, Blut, vaginales Sekret oder Urin der Patientin sein.

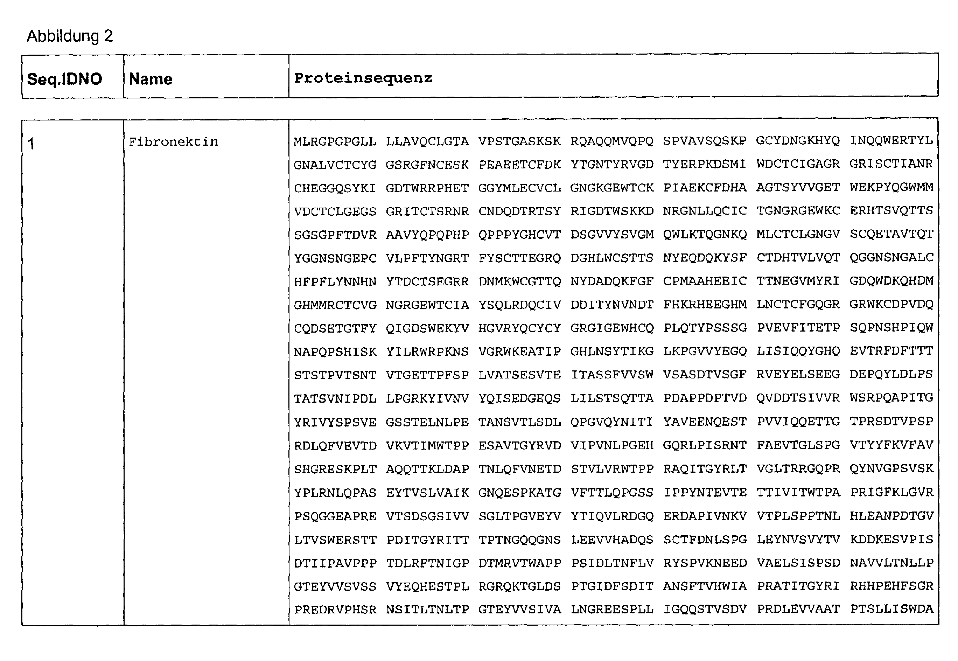

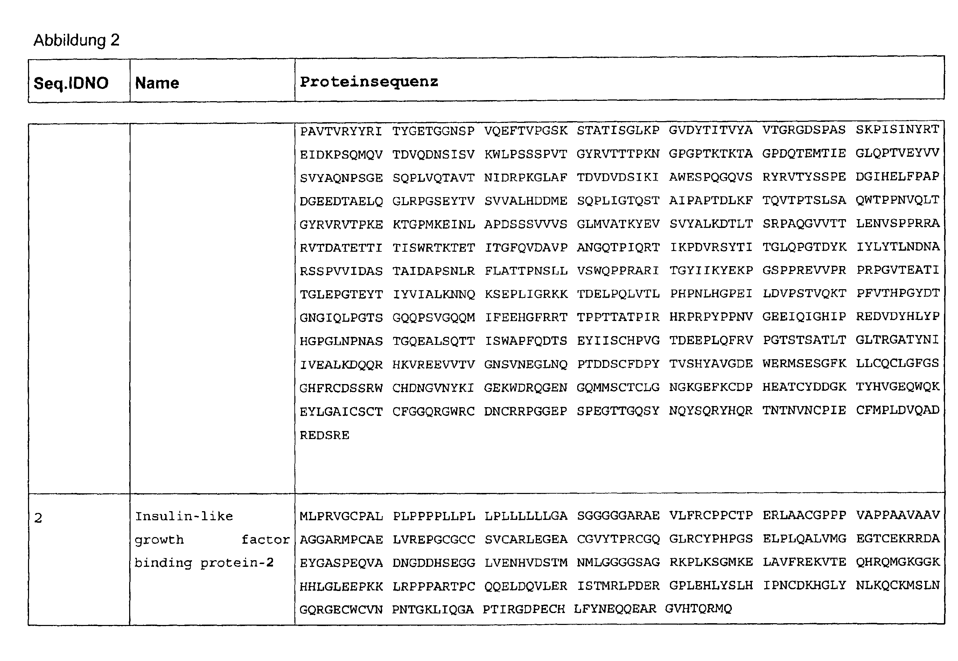

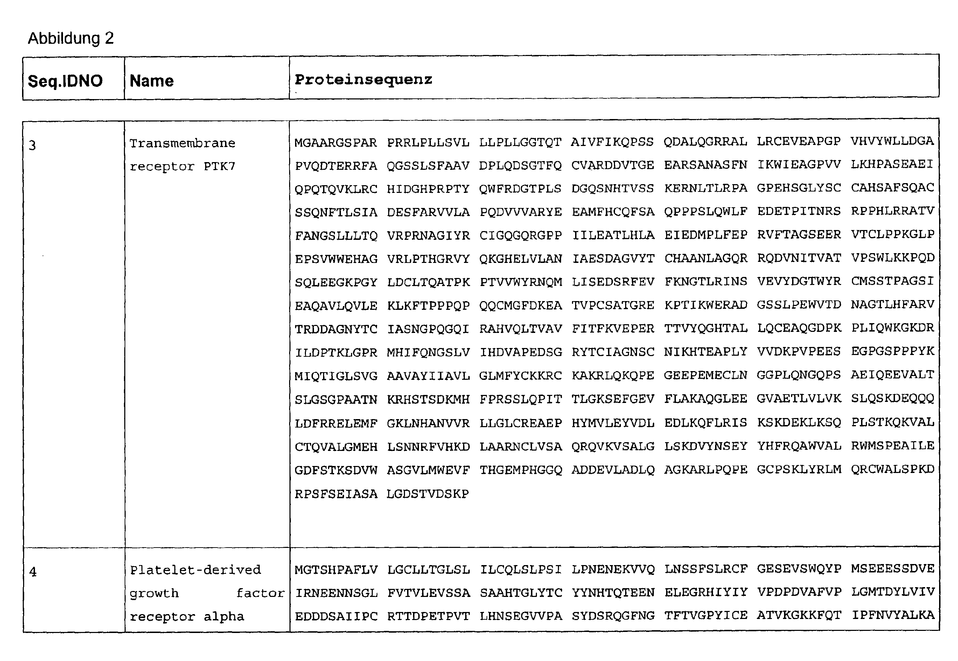

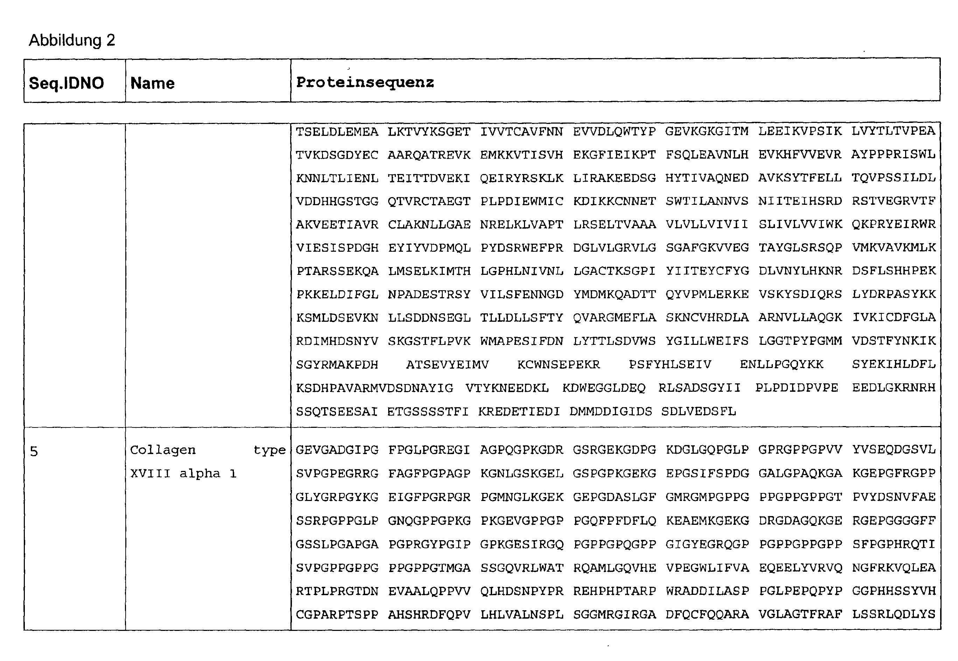

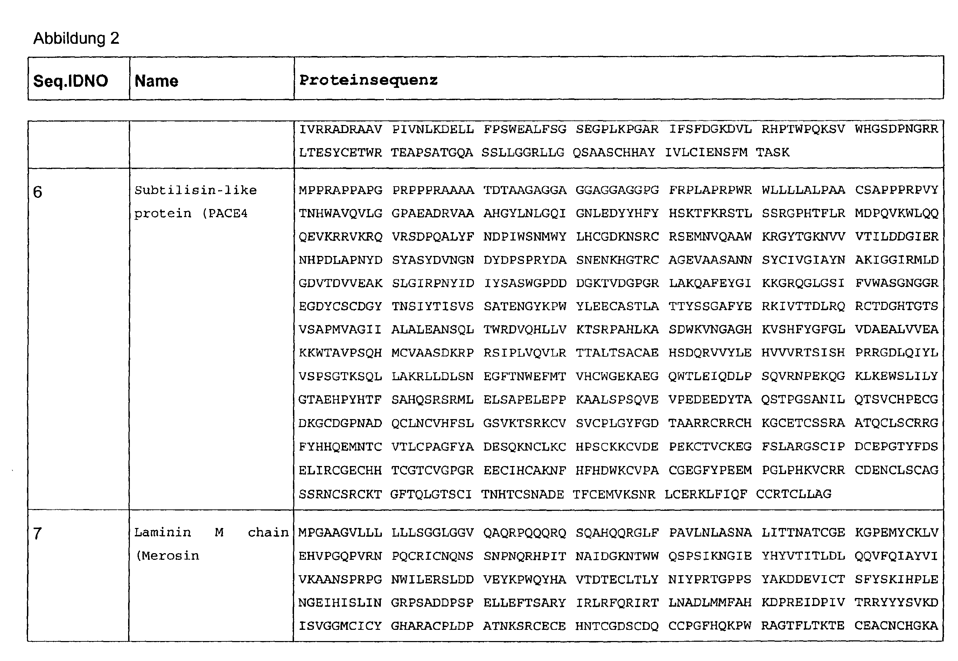

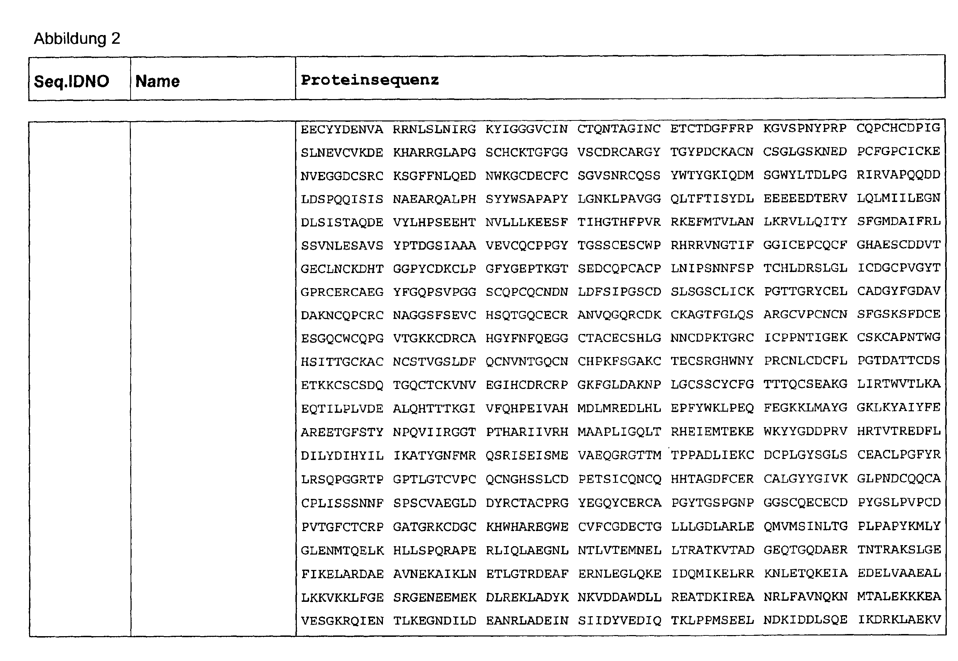

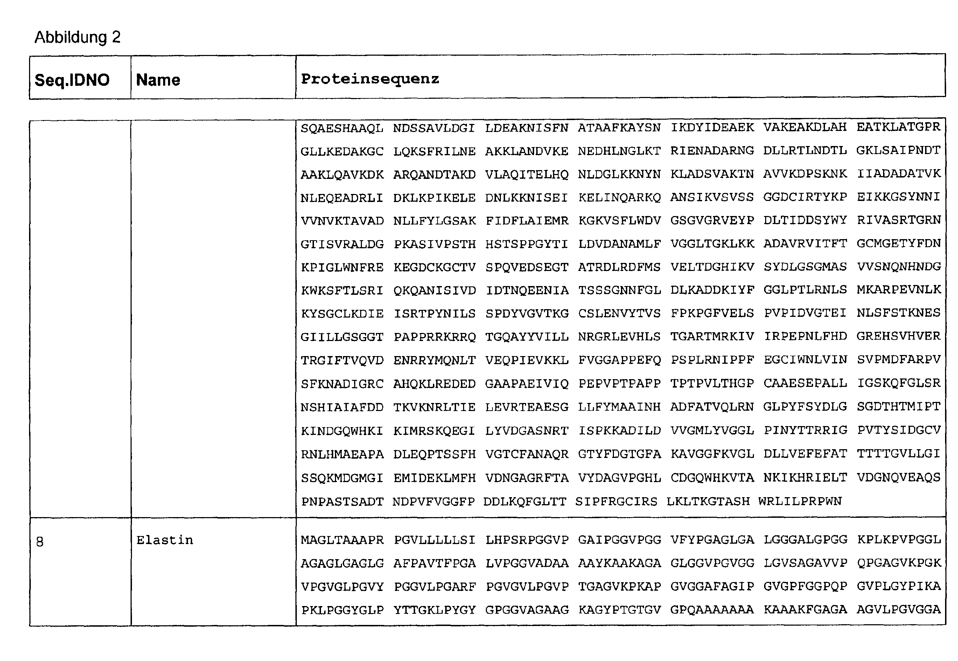

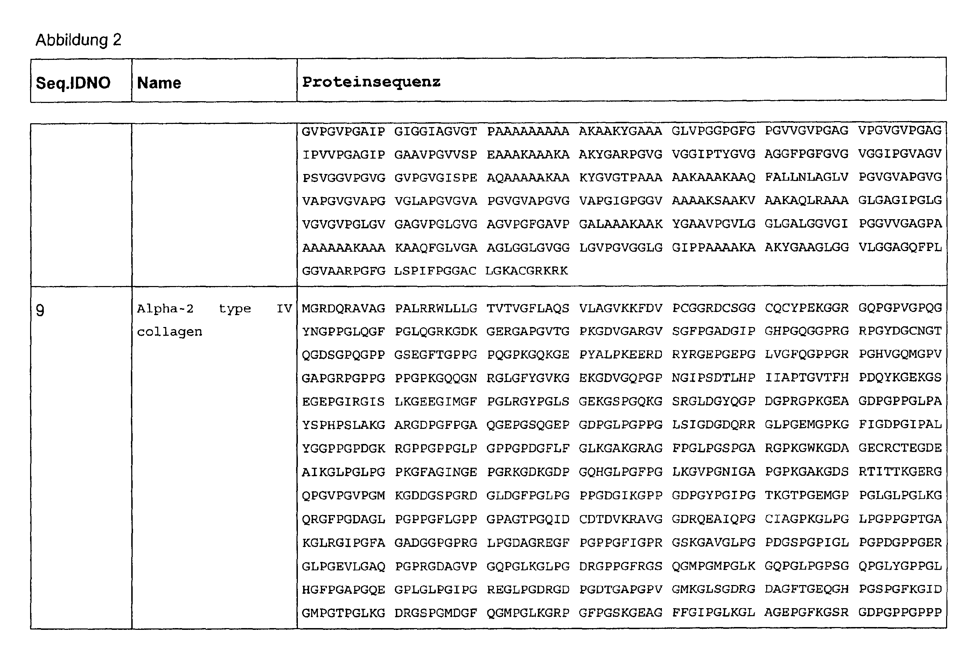

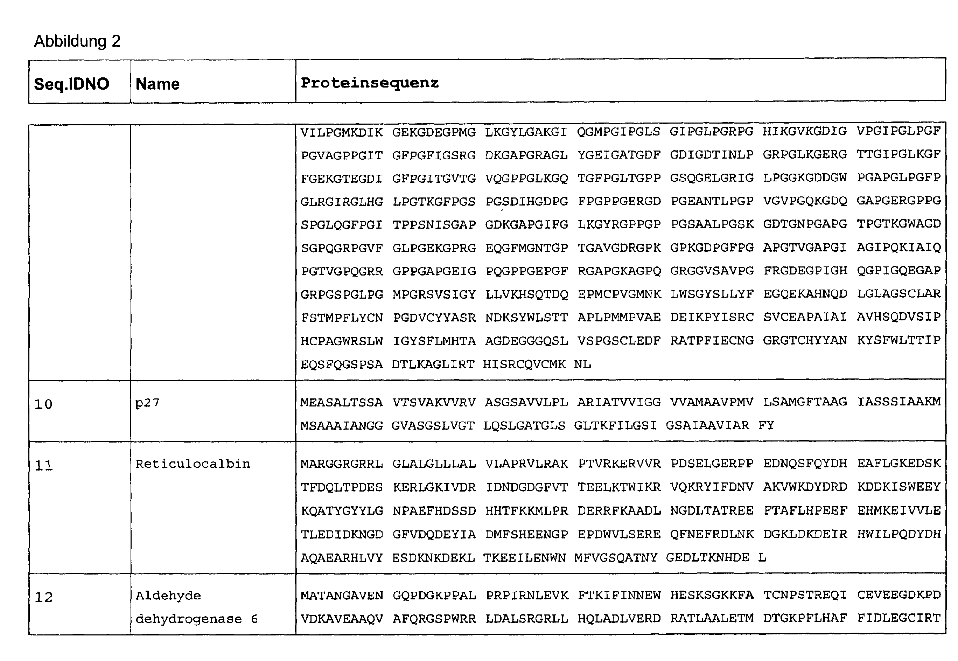

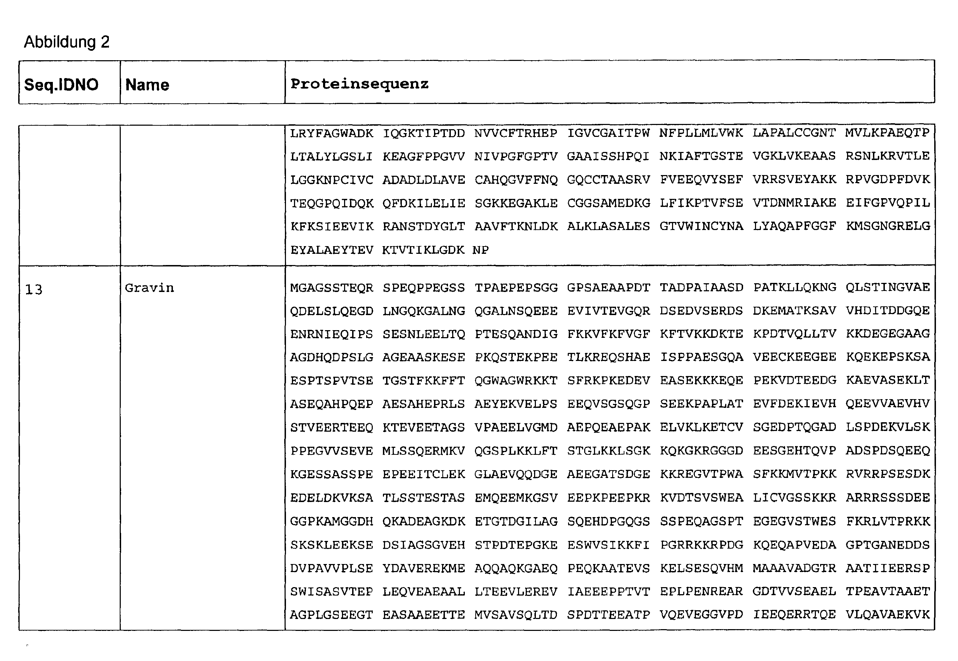

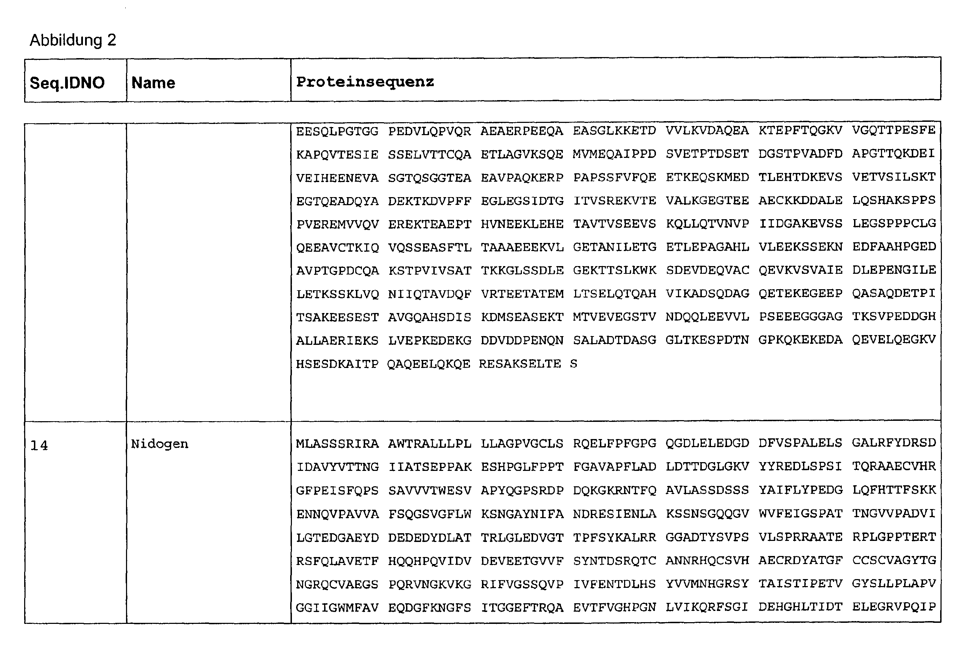

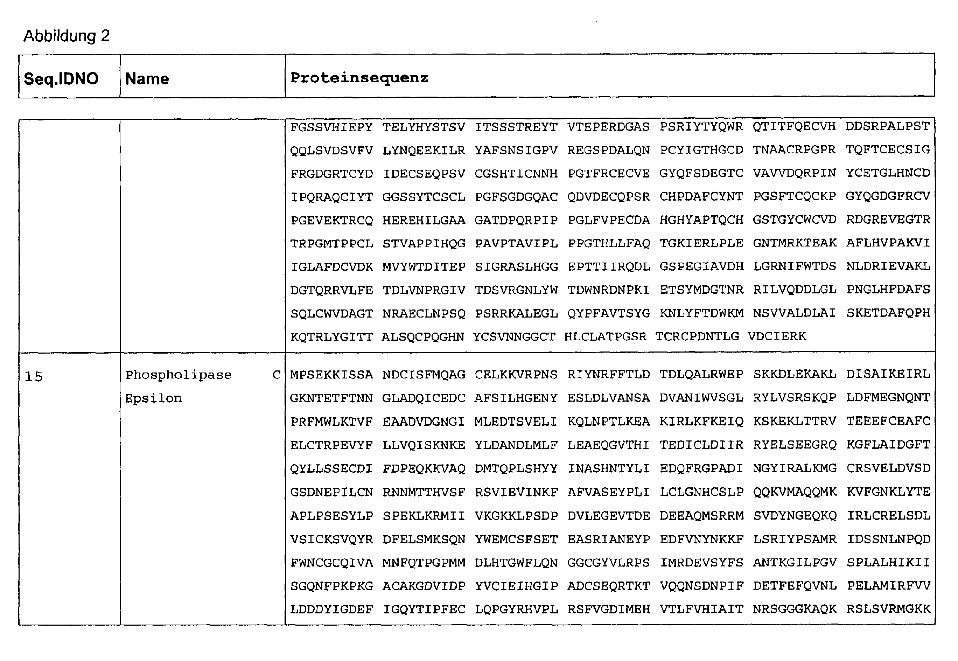

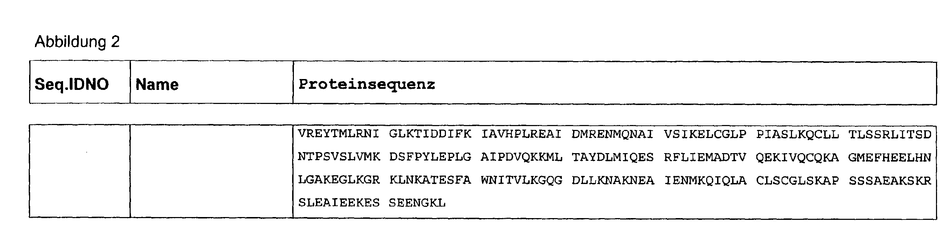

Ein Genprodukt ist entweder mRNA, die daraus abgeleitete cDNA, ein Polypeptid oder Teile eines Polypeptids. Die Aminosäuresequenzen der Polypeptide sind in Abbildung 2 dargestellt.

Die erfindungsgemäße Methode kann zur erstmaligen Diagnose von Endometriose eingesetzt werden. In diesem Fall wird die Menge des Genproduktes in der Patientinnenprobe mit einer Kontrollprobe von nicht erkrankten Frauen verglichen. Die erfindungsgemäße Methode kann auch zur Beurteilung des Verlaufs der Krankheit verwendet werden. So kann z.B. der Erfolg einer Therapie bestimmt werden. In diesem Fall wird die Patientinnenprobe mit einer älteren Probe derselben Patientin verglichen.

Das Genprodukt Polypeptid oder ein Teilstück eines Polypeptids wird durch Immunassays detektiert. Dazu werden spezifische Antikörper gegen eines oder mehrere Polypeptide ausgewählt aus der in Abbildung 2 beschriebenen Gruppe, hergestellt. Die Antikörper können monoklonal oder polyklonal sein. Sie können gegen jeweils das gesamte Polypeptid oder gegen Fragmente davon gerichtet sein. Die Gewinnung eines solchen Antikörpers erfolgt nach Standardmethoden durch Immunisierung von Versuchstieren. Die Antikörper werden dann z.B. in einem ELISA (enzyme-linked-immunosorbent assay), in einem RIA (radioimmunoassay) oder in der Immunhistochemie zur Bestimmung der Menge des Genproduktes verwendet (Aoki, K. et al. 1996; Forensic Sci. Int. 80, 163-173).

Die Erfindung betrifft ferner die Verwendung eines erfindungsgemäßen Antikörper-Chips zur Diagnose von Endometriose. Antikörper-Chips sind miniaturisierte Träger, meist aus Glas oder Silizium, auf deren Oberfläche Antikörper bekannter Spezifität in einem geordneten Raster in hoher Dichte immobilisiert werden. Die Detektion der Protein/Protein Interaktionen kann durch Massenspektrometrie, Fluoreszenz oder surface plasmon resonance erfolgen. Es können Antikörper, welche die aus der in Abbildung 2 beschriebenen Gruppe ausgewählten Proteine spezifisch binden, auf dem Antikörper-Chip immobilisiert sein. Methoden zur Herstellung und Verwendung von Antikörper-Chips sind in Kreider BL, Med Res Rev 2000,20:212-215 beschrieben.

Die Genprodukte mRNA bzw. die daraus abgeleitete cDNA können durch Hybridisierung mit Oligonukleotiden, z.B. durch einen Northern blot bestimmt werden. Diese Oligonukleotide haben Sequenzen, die komplementär zu Teilsequenzen des zu detektierenden Genprodukts sind, und können z.B. mit einer chromogenen, radioaktiven oder fluoreszierenden Gruppe markiert sein. Vor der Hybridisierung kann die cDNA mit Hilfe der PCR amplifiziert werden (Sambrook, J. et al. 1989; Cold Spring Harbor Laboratory Press).

Die Genprodukte mRNA bzw. die daraus abgeleitete cDNA können auch durch quantitative PCR (polymerase chain reaction) bestimmt werden.

Die mRNA kann auch durch *in situ* Hybridisierung mit antisense-RNA bestimmt werden. Dabei kann die antisense-RNA mit Dioxigenin, ³²P oder ³³P markiert sein. Antisense Nukleinsäure ist eine DNA und/oder RNA , die komplementär zu einer mRNA ist. Sie kann die gesamte komplementäre Sequenz oder Teilsequenzen umfassen. Diese Methode ist dem Fachmann bekannt (Barlati, S. et al. 1999; Histol. Histopathol. 14, 1231-1240).

Die Hybridisierung kann auch mit Hilfe eines DNA-Chips erfolgen. Die Erfindung betrifft daher weiterhin einen DNA - Chip, auf dem mindestens ein Oligonukleotid immobilisiert ist, das der vollständigen cDNA-Sequenz oder einer Teilsequenz bzw. komplementären Sequenz eines Genes ausgewählt aus der Gruppe, die in Abbildung 1 beschrieben ist, entspricht. Die Erfindung betrifft somit ferner die Verwendung eines erfindungsgemäßen DNA-Chips zur Diagnose von Endometriose.

DNA-Chips, auch als DNA-Mikroarrays bekannt, sind miniaturisierte Träger, meist aus Glas oder Silizium, auf deren Oberfläche DNA-Moleküle bekannter Sequenz in einem geordneten Raster in hoher Dichte immobilisiert werden. Die Oberflächengebundenen DNA-Moleküle werden mit komplementären, eventuell markierten Nukleinsäuren hybridisiert. Die Markierung kann ein Fluoreszenzfarbstoff sein.

Bei Oligonukleotid-Chips stellen die Oligonukleotide, die auf einem erfindungsgemäßen DNA-Chip gebunden sein können, Teilsequenzen der Genprodukte (mRNA bzw. daraus abgeleitete cDNA) in der Sense- oder Antisense-Richtung dar. Es können ein oder mehrere Oligonukleotide pro Gen auf dem DNA-Chip gebunden sein. Bevorzugt sind 25 Nukleotid-lange Oligonukleotide, die aus dem nicht kodierenden Strang abgeleitet sind. Diese werden bevorzugt aus dem jeweiligen 3'untranslatierten Ende des Gens ausgewählt. Zur Detektion können Oligonukleotide von einem Gen, mehreren Genen oder allen Genen ausgewählt aus der in Abbildung 1 beschriebenen Gruppe eingesetzt werden. Methoden zur Herstellung und Verwendung von DNA-Chips sind z.B. in den US-Patenten Nr. 5,578,832; 5,556,752 und 5,510,270 beschrieben.

Bei cDNA Chips sind die vollständigen Genprodukte (cDNAs) oder Subfragmente (200-500bp lang) auf dem Chip gebunden. Die Methode wird z. B. in Eckmann, L. et al., J Biol Chem 2000, 275: 14084-14094 beschrieben.

Das Genprodukt mRNA kann auch durch chromogene Assays bestimmt werden.

### Beschreibung der Abbildungen

Abb. 1 zeigt die Liste der Gene, die in der sekretorischen Phase bei Vorliegen einer Endometriose herunterreguliert sein können und damit für eine Diagnostik der Endometriose verwendet werden können. In Spalte 1 sind die Namen und die Datenbank-Nummer (Accession Numbers) der Gene aufgelistet, die bei der Analyse als differentiell reguliert gefunden wurden. In Spalte 2 findet sich der Vergleich von Proben aus der sekretorischen Phase (sekr. Phase), jeweils *Endometriose* versus *Normal* (keine Endometriose); *down* bezeichnet den Zustand der Herunterregulation. Die erste Zahl in Klammern gibt an, wie oft das Gen als heraufreguliert und die zweite Zahl gibt an, wie oft das Gen als herunterreguliert gefunden wurde. Für diese Analyse wurden 20 Einzelvergleiche durchgeführt. In Spalte 3 findet sich der Vergleich von Proben aus der proliferativen Phase (prol. Phase). Für diese Analyse wurden 30 Einzelvergleiche durchgeführt. Die Bezeichnung *down* beschreibt den gleichen Zustand wie in Spalte 1, *nc* bedeutet no *correlation* (keine Korrelation), d.h. man findet dieses Gen sowohl herunter- als auch heraufreguliert. Die Bedeutung der Zahlen ist analog zu Spalte 2. In der vierten Spalte findet sich der Vergleich von Proben aus der sekretorischen Phase mit Proben aus der proliferativen Phase. Hier wurde Endometrium von Frauen ohne Endometriose miteinander verglichen. Für diese Analyse wurden 25 Einzelvergleiche durchgeführt. Die Bezeichnung up beschreibt den Zustand der Heraufregulation. Die Bedeutung der Zahlen ist analog zu Spalte 2.

Abbildung 2 zeigt eine Liste der Polypeptide, die von den in Abbildung 1 dargestellten Genen kodiert werden und bei Vorliegen einer Endometriose vermindert exprimiert werden.

### Beispiele

Die in den Beispielen verwendeten molekularbiologischen Methoden wie z.B. Isolierung von RNA, Sequenzierung von DNA, RNAse Protection, Northern Blot Analyse, Polymerase-Kettenreaktion (PCR) wurden nach Standardprotokollen, wie in bekannten Lehrbüchern wie z.B. in Molecular Cloning, A Laboratory Manual (Sambrook, J. et al. 1989; Cold Spring Harbor Laboratory Press) beschrieben, durchgeführt. Methoden für Subtraktionsanalysen der Genexpression sind z.B. in Liang, P. und Pardee, A. B. 1995; Curr. Opin. Immunol. 7, 274-280 beschrieben.

### Beispiel 1: Identifizierung von Endometriose-assoziierten Genen

Gene, die mit dem Krankheitsbild der Endometriose assoziiert sind, wurden durch Vergleich von Endometriumproben folgender Patientinnengruppen identifiziert:
1. Proliferative Phase: Tage 4-14 nach der letzten Menstruation. Diese Gruppe setzte sich aus Patientinnen zusammen, bei denen aufgrund von Leiomyomen eine Hysteroskopie oder Hysterektomie durchgeführt wurde.
2. Sekretorische Phase: Tage 15-28 nach der letzten Menstruation. Diese Gruppe setzte sich aus Patientinnen zusammen, wie unter 1. beschrieben.
3. Proliferative Phase plus Endometriose: Tage 4-14 nach der letzten Menstruation. Die Patientinnen dieser Gruppe litten an Endometriose.
4. Sekretorische Phase plus Endometriose: Tage 15-28 nach der letzten Menstruation. Die Patientinnen dieser Gruppe litten an Endometriose.

Endometrium von Frauen mit Endometriose wurde mittels einer Strichcurettage gewonnen. Das Endometrium der Vergleichsgruppe wurde von Frauen im Rahmen einer Hysteroskopie oder einer Hysterektomie, die wegen eines Leiomyoms vorgenommen wurde, gewonnen. Das Gewebe wurde nach der Entnahme in flüssigem Stickstoff tiefgefroren. Anschließend wurde Gesamt-RNA aus den Proben extrahiert. Diese RNA wurde amplifiziert, durch einen Fluoreszenzmarker markiert, und mit einem DNA-Chip (Human SL array der Firma Affymetrix, enthaltend Oligonukleotide für ca. 7000 humane Gene) hybridisiert. Nach dem Hybridisierungsverfahren wurde der DNA-Chip in einem Scanner analysiert. Die Hybridisierungsmuster aller Gensequenzen, die sich auf dem Chip befinden, wurden zwischen allen Proben verglichen. Insgesamt wurden 20 Einzelvergleiche mit Proben aus der sekretorischen Phase und 30 Einzelvergleiche mit Proben aus der proliferativen Phase durchgeführt bei denen jeweils eine Probe von einer Frau mit Endometriose stammte und eine Probe von einer Frau, die nicht an Endometriose litt.

Als differentiell reguliert betrachtet wurden alle diejenigen Gene, die in mindestens der Hälfte der Fälle (10 Vergleiche) um mindestens den Faktor 1.5 gegenüber der Kontrollgruppe (Proben von Frauen ohne Endometriose) herauf- oder herunterreguliert waren. Außerdem wurden 25 Einzelvergleiche von Proben aus der sekretorischen Phase mit Proben aus der proliferativen Phase durchgeführt. Hier wurde Endometrium von Frauen ohne Endometriose verglichen.

Die Ergebnisse sind in Abbildung 1 dargestellt. Die aufgelisteten Gene können als Differenzierungsmarker betrachtet werden. Ausgehend von der Betrachtung, daß die proliferative Phase, dem Namen entsprechend von proliferativen Prozessen dominiert wird, wird die sekretorische Phase eher als Differenzierungsphase angesehen. Vor diesem Hintergrund sollten Gene, die für die Differenzierung von Bedeutung sind, während dieser Phase heraufreguliert werden (vergleiche mit Abbildung 1, Spalte 4) und während der proliferativen Phase herunterreguliert oder gleichbleibend reguliert sein (vergleiche mit Abbildung 1, Spalte 3). Die Gene, die in Spalte 1 aufgelistet sind, erfüllen diese Kriterien und werden daher als Differenzierungsmarker bezeichnet. Dass diese Gene bei Frauen mit Endometriose herunterreguliert sind (Spalte 2), deutet auf eine gestörte Differenzierung in der sekretorischen Phase hin.

### Beispiel 2: Diagnostik von Endometriose

### 1. Probengewinnung

Für die DNA-Chip-Analyse wird Endometriumgewebe aus Patientinnen gewonnen und Gesamt-RNA daraus isoliert. Die RNA wird dann amplifiziert und an einen Fluoreszenzmarker gekoppelt. Für den Immuntest kann Peritonealflüssigkeit, Blut, Vaginal-Sekret, Urin oder Endometriumgewebe von der Patientin gewonnen werden.

### 2. Detektion der Genprodukte

### 2a. mit Hilfe eines DNA-Chips

Zuerst werden die geeigneten DNA-Sequenzen aus den Genen, die aus der in Abbildung 1 beschriebenen Gruppe ausgewählt werden, bestimmt. Geeignet sind Sequenzen, die mit den ausgewählten Gentranskripten hybridisieren können. Die Oligonukleotide werden dann durch einen chemischen Prozess, der auf photolithographischen Verfahren basiert, auf dem Chip hergestellt. Dazu werden photolithographische Masken verwendet, die durch geeignete Computer-Algorithmen erzeugt wurden.

Die markierte RNA wird mit dem Chip in einem Hybridisierungsofen inkubiert. Anschliessend wird der Chip in einem Scanner analysiert, der die Hybridisierungsprofile bestimmt. Dadurch kann festgestellt werden, ob in der sekretorischen Phase eines oder mehrere der Gene der in Abbildung 1 aufgelisteten Gene runterreguliert ist, was auf eine Endometriose hinweist.

### 2b. durch Immuntest

Für die Durchführung eines Immuntestes benötigt man spezifische Antikörper, die an die in Abbildung 2 beschriebenen Polypeptide binden. Die Antikörper können mono- oder polyklonale Antikörper sein, die gegen die aufgereinigten Proteine, Peptide, ausgewählt aus den kodierten Proteinen, oder rekombinant hergestellte Fragmente oder Gesamtprotein gerichtet sind.

Erfolgt die Analyse mittels Immunohistochemie verwendet man Endometrium, das der zu analysierenden Patientin entnommen wurde. Nach geeigneter Fixierung des Gewebes, z.B. mittels Formaldehyd und anschließender Einbettung in Paraffin kann das Gewebe für die immunhistochemische Analyse verwendet werden. Dazu werden von dem fixierten und eingebetteten Gewebe mit einem Mikrotom Schnitte geeigneter Dicke, z.B. 4 µm, angefertigt. Der oder die spezifischen Antikörper werden dann mit den weiter vorbereiteten Gewebeschnitten (z.B. Deparaffinierung, Blocken) eine Zeitlang unter geeigneten Temperaturbedingungen, z.B. 1h bei Raumtemperatur inkubiert. Nach Waschschritten mit einer geeigneten Lösung, z.B. PBS, werden die Schnitte in einem 2. Schritt mit einem geeigneten zweiten Antiköper inkubiert, der für die nachfolgenden Reaktionen, z.B. biotiniliert ist. Der 2. Antiköper bindet an die für die jeweilige Spezies konstante Region des 1. Antiköpers. Nach geeigneter Inkubationszeit und Waschschritten wird nun in einem dritten Schritt die Gewebeprobe z.B. mit Horse-Redish Peroxidase, gekoppelt an Streptavidin, inkubiert. Nach geeigneter Inkubationszeit und Waschschritten wird nun in einem letzten Schritt durch Zugabe eines geeigneten Farbstoffs, z.B. DAB, von der Peroxidase eine Enzymreaktion katalysiert, die zu einer Farbreaktion dort führt, wo der 1. Antikörper spezifisch gebunden hat. Nach Abstoppen der Enzymreaktion und Waschschritten kann der Gewebeschnitt nun getrocknet, fixiert und mit einem Deckgläschen versehen, unter dem Mikroskop analysiert werden. Um zu entscheiden, ob in der Gewebeprobe ein quantitativer oder auch qualitativer Unterschied besteht, muß eine entsprechende Kontrolle einer Probe von einer Frau ohne Endometriose als Vergleich herangezogen werden.

Erfolgt die Analyse mittels Westernblot werden die gewonnenen Gewebeproben oder Extrakte aus der Peritonealflüssigkeit, Blut, Vaginal-Sekret oder Urin mittels einer Polyacrylamid-Gelelektrophorese aufgetrennt. Nach der Auftrennung werden die in dem Gel aufgetrennten Polypeptide durch Anlegen eines elektrischen Stroms auf eine geeignete Trägermembran, z.B. Nitrozellulose, überführt. Die auf der Trägermembran fixierten Proteine werden nun in einem 1. Schritt mit dem oder den spezifischen Antikörpern inkubiert. Nach geeigneten Waschvorgängen, z.B. mit TBS/TBST, wird die Trägermembran in einem 2. Schritt mit einem 2. Antikörper inkubiert, der an die für die jeweilige Spezies konstante Region des 1. Antiköpers bindet. Der 2. Antikörper kann eine radioaktive Markierung tragen oder ein gekoppeltes Enzym, z.B. alkalische Phosphatase, die in einer nachfolgenden Farbreaktion ein farbloses Substrat in ein farbiges Substrat umwandelt. Da die Menge des an dem Antigen gebundenen des 2. Antikörpers derjenigen des Antigens proportional ist, kann daher die Menge des gemessenen Farbstoffs für eine quantitative Analyse des oder der in dem Extrakt vorhandenen Polypeptide genutzt werden.

Erfolgt die Analyse mittels eines Festphasenimmunoassays wird der oder die spezifischen Antikörper an eine polymere Trägermatrix, z.B. Polyvinylchlorid, gebunden. Anschließend inkubiert man den oder die fixierten Antikörper mit dem Extrakt, der aus z.B. aus der Peritonealflüssigkeit, Blut, Vaginal-Sekret oder Urin gewonnen wurde. Nach geeigneten Waschvorgängen wird in einem 2. Schritt ein 2. Antikörper hinzugegeben, der an einer anderen Stelle des zu detektierenden Antigens spezifisch bindet. Der 2. Antikörper trägt z.B. eine radioaktive oder Fluoreszenzmarkierung und kann daher in einem 3. Schritt hochempfindlich nachgewiesen werden. Die an dem Antigen gebundene Menge des 2. Antikörpers ist derjenigen des Antigens proportional und kann daher für eine quantitative Analyse des oder der in dem Extrakt vorhandenen Proteine genutzt werden.

Erfolgt die Analyse mittels ELISA (enzyme linked immunosorbent assay) wird der oder die spezifischen Antikörper an eine polymere Trägermatrix, z.B. Polyvinylchlorid, gebunden. Anschließend inkubiert man den oder die fixierten Antikörper mit dem Extrakt, der aus z.B. aus der Peritonealflüssigkeit, Blut, Vaginal-Sekret oder Urin gewonnen wurde. Nach geeigneten Waschvorgängen wird in einem 2. Schritt ein 2. Antikörper hinzugegeben, der an einer anderen Stelle des zu detektierenden Antigens spezifisch bindet. Der 2. Antikörper trägt zusätzlich noch ein gekoppeltes Enzym, z.B. eine alkalische Phosphatase. Dieses Enzym katalysiert nun in einem nachfolgenden Schritt die Umwandlung eines farblosen Substrats in ein farbiges Produkt. Es kann aber auch ein nicht-fluoreszierendes Substrat in ein fluoreszierendes Substrat umgewandelt werden. Die Menge des farbigen oder fluoreszierenden Produkts kann kolorimetrisch gemessen werden. Da die Menge des an dem Antigen gebundenen 2. Antikörpers derjenigen des Antigens proportional ist, kann daher die Menge des gemessenen Farbstoffs oder Fluoreszenzproduktes für eine quantitative Analyse des oder der in dem Extrakt vorhandenen Polypeptide genutzt werden.

## Patentansprüche

1. Methode zur in vitro Diagnose von Endometriose, **dadurch gekennzeichnet, daß** die Menge an Genprodukt von mindestens einem Gen aus der Gruppe bestehend aus Fibronectin, Insulin-like growth factor binding protein-2, Transmembrane receptor PTK7, Platelet-derived growth factor receptor alpha, Collagen type XVIII alpha 1, Subtilisin-like protein (PACE4), Laminin M chain (Merosin), Elastin, Collagen type IV alpha 2, p27 interferon alpha-inducible gene, Reticulocalbin, Aldehyde Dehydrogenase 6, Gravin, Nidogen und Phospholipase C Epsilon in einer Patientinnenprobe bestimmt wird und mit der Menge an diesem Genprodukt in einer Kontrollprobe verglichen wird, wobei eine geringere Menge an diesem Genprodukt auf das Vorliegen einer Endometriose hinweist.

2. Verwendung von Antikörpern gegen ein oder mehrere Proteine kodiert von Genen aus der Gruppe bestehend aus Fibronectin, Insulin-like growth factor binding protein-2, Transmembrane receptor PTK7, Platelet-derived growth factor receptor alpha, Collagen type XVIII alpha 1, Subtilisin-like protein (PACE4), Laminin M chain (Merosin), Elastin, Collagen type IV alpha 2, p27 interferon alpha-inducible gene, Reticulocalbin, Aldehyde Dehydrogenase 6, Gravin, Nidogen und Phospholipase C Epsilon oder gegen Teile des Polypeptids oder der Proteine zur Diagnose von Endometriose.

3. DNA - Chip, **dadurch gekennzeichnet, daß** auf dem Chip mindestens ein Oligonukleotid, das eine Teilsequenz einer DNA ausgewählt aus der Gruppe bestehend aus Fibronectin, Insulin-like growth factor binding protein-2, Transmembrane receptor PTK7, Platelet-derived growth factor receptor alpha, Collagen type XVIII alpha 1, Subtilisin-like protein (PACE4), Laminin M chain (Merosin), Elastin, Collagen type IV alpha 2, p27 interferon alpha-inducible gene, Reticulocalbin, Aldehyde Dehydrogenase 6, Gravin, Nidogen und Phospholipase C Epsilon oder deren komplementären Sequenz umfaßt, gebunden ist.

4. Verwendung eines DNA-Chips nach Anspruch 3 zur Diagnose von Endometriose.
